# EUROPÄISCHE PATENTANMELDUNG

(11) **EP 1 505 056 A1**
(43) Veröffentlichungstag der Anmeldung: **09.02.2005**
(21) Anmeldenummer: 04017619.0
(22) Anmeldetag: 26.07.2004
(51) Int. Cl.: C07C 209/40, C07C 209/88, C07C 211/29

(54) **Enantiomerenangereicherte 1-Phenylethylamine**

(30) Priorität: 07.08.2003 DE 10336185
(71) Anmelder: Bayer Chemicals AG, 51368 Leverkusen (DE)
(72) Erfinder: Schlummer, Björn, Dr., 51377 Leverkusen (DE); Dreisbach, Claus, Dr., 42799 Leichlingen (DE); Cotté, Alain, Dr., 51377 Leverkusen (DE)

(57) **Zusammenfassung**

Die vorliegende Erfindung betrifft ein Verfahren zur Herstellung von nitrosubstituierten, enantiomerenangereicherten 1-Phenylethylaminen sowie ihre Verwendung.

## Beschreibung

Die vorliegende Erfindung betrifft ein Verfahren zur Herstellung von nitrosubstituierten, enantiomerenangereicherten 1-Phenylethylaminen sowie ihre Verwendung.

Nitrosubstituierte, enantiomerenangereicherte 1-Phenylethylamine sind wichtige Zwischenprodukte für die Synthese von pharmazeutischen Wirkstoffen wie insbesondere IMPDH-Inhibitoren (siehe auch WO-A 00/56331). Im Allgemeinen kann die Darstellung von nitrosubstituierten 1-Phenylethylaminen beispielsweise über die reduktive Aminierung von nitrosubstituierten Acetophenonen erfolgen.

So ist beispielsweise aus F. Nerdel, H. Liebig, *Liebigs Ann. d*. *Chemie* **1954**, *87*, 221-222 bekannt, 1-(3-Nitrophenyl)ethylamin-Hydrochlorid durch Umsetzung von 3-Nitroacetophenon mit Ammoniumcarbonat und Ameisensäure bei 190°C herzustellen.

A. de Roocker, P. de Radzitzky, *Bull.Soc.Chim.Belg* 1963, 202-207 beschreiben die Darstellung von 1-(3-Nitrophenyl)ethylamin durch Umsetzung von 3-Nitroacetophenon mit Ammoniumcarbonat und Ameisensäure.

Nachteilig an den genannten Verfahren sind die hohen Reaktionstemperaturen, denen die eingesetzten Nitroverbindungen ausgesetzt werden, die insbesondere im technischen Maßstab zu erheblichen Sicherheitsrisiken führen können.

Alternativ zur Reduktion mit Ameisensäure sind auch Verfahren bekannt, in denen die Reduktion von nitrosubstituierten Acetophenonen mit Natriumcyanoborhydrid durchgeführt wird. J. L. Kelley, E. W. McLean, R. M. Ferris, J. L. Howard, *J*. *Med*. *Chem*. **1990,** *33*, 1910-1914 beschreiben beispielsweise die Darstellung von 1-(3-Nitrophenyl)ethylamin durch Umsetzung von 3-Nitroacetophenon mit Ammoniumacetat und Natriumcyanoborhydrid in Methanol bei 25°C.

Durch die sehr langen Reaktionszeiten und die Verwendung des teuren und giftigen Reduktionsmittels Natriumcyanoborhydrid ist jedoch auch dieses Verfahren technisch nicht attraktiv.

Eine weitere Darstellungsmöglichkeit für nitrosubstituierte 1-Phenylethylamine ist die Reduktion von nitrosubstituierten 1-Azido-1-phenylethanen mit Triphenylphosphin, wie in WO-A 00/56331 beschrieben. Für die technische Synthese verbietet sich jedoch der Einsatz von Aziden aus sicherheitstechnischen Aspekten.

Es bestand daher weiterhin das Bedürfnis, ein Verfahren bereitzustellen, über das nitrosubstituierte, enantiomerenangereicherte 1-Phenylethylamine in einfacher und effizienter Weise zugänglich sind.

Es wurde nun ein Verfahren zur Herstellung von enantiomerenangereicherten Verbindungen der Formel (I) gefunden 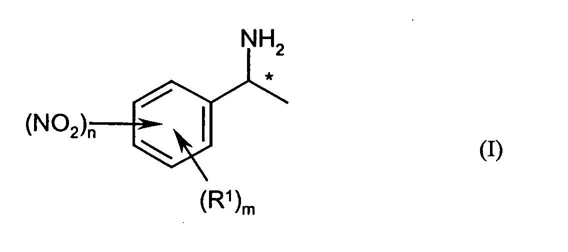 in der
* ein stereogenes Kohlenstoffatom markiert
   n für 1, 2 oder 3 steht,
   m für eine ganze Zahl zwischen 0 und (5-n) steht und
   R¹ jeweils unabhängig ausgewählt ist aus den Resten: C₁-C₁₂-Alkyl, C₁-C₁₂-Alkoxy, C₁-C₁₂-Alkylthio, C₅-C₁₄-Aryl, C₅-C₁₄-Aryloxy, C₆-C₁₅-Arylalkyl, C₆-C₁₅-Arylalkoxy, Chlor, Fluor, Cyano, freies oder geschütztes Formyl, freies oder geschütztes Hydroxy, freies oder geschütztes Mercapto, C₁-C₁₂-Halogenalkyl, C₁-C₁₂-Halogenalkylthio, C₁-C₁₂-Halogenalkoxy und Resten der Formeln (IIa) und (IIb),

      A-B-D-E (IIa)

      A-E (IIb)
   in denen
   - A: fehlt oder für einen C₁-C₈-Alkylen-, C₁-C₈-Alkenylen- oder C₁-C₈-Halogenalkylenrest steht und
   - B: fehlt oder für Sauerstoff, Schwefel oder NR² steht, wobei
   R² für Wasserstoff, C₁-C₈-Alkyl, C₆-C₁₅-Arylalkyl oder C₅-C₁₄-Aryl steht und
   - D: für eine Carbonyl-Gruppe steht und
   - E: für OR³ oder N(R⁴)₂ steht,
   wobei
   - R³: für C₁-C₈-Alkyl, C₆-C₁₅-Arylalkyl oder C₅-C₁₄-Aryl und
   - R⁴: jeweils unabhängig für Wasserstoff, C₁-C₈-Alkyl, C₆-C₁₅-Arylalkyl oder C₆-C₁₄-Aryl steht oder N(R⁴)₂ zusammen für einen cyclischen Aminorest mit 4 bis 12 Kohlenstoffatomen steht und
   das dadurch gekennzeichnet ist, dass

- in einem Schritt A)
   Verbindungen der Formel (III) 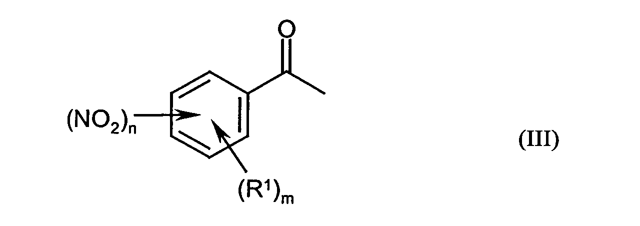 mit Verbindungen der Formel (IVa) oder (IVb)

   R⁵O-NH₂ (IVa)

   R⁵O-NH₂ • HX (IVb)

   in denen
   R⁵ für Wasserstoff oder C₁-C₁₂-Alkyl steht und
   X für ein Anion steht
   zunächst zu Verbindungen der Formel (V) 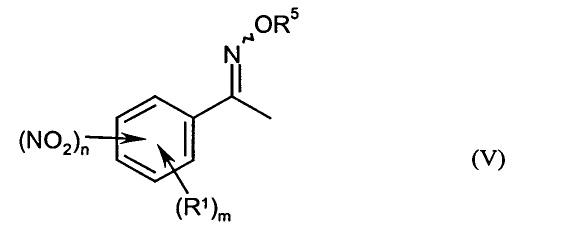 umgesetzt werden und
- in einem Schritt B)
   die Verbindungen der Formel (V)
   - in einem organischen Lösungsmittel
   - mit einem komplexen Borhydrid und
   - einer Säure zu racemischen Verbindungen der Formel (Ia) 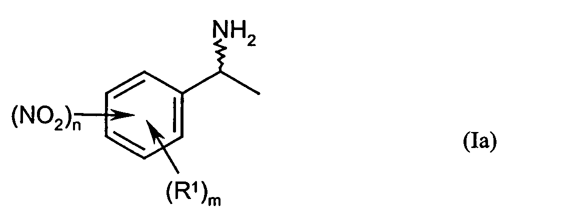 reduziert werden und
      in einem Schritt C)
      die racemischen Verbindungen der Formel (Ia) mit Hilfe von enantiomerenangereicherten Säuren enantiomerenangereichert werden.
      Gegebenenfalls kann als weiterer Schritt D)
      - die Umsetzung der enantiomerenangereicherten Verbindungen der Formel (I) zu enantiomerenangereicherten Ammoniumsalzen der Formel (Ib) erfolgen.

Dabei steht in Formel (Ib) 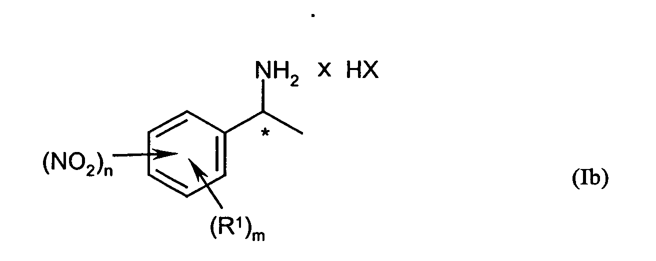
- X: für ein Halogenid oder ein Sulfonat.

Bevorzugte Verbindungen der Formel (I) sind solche in denen
- n: für 1 steht,
- m: für 0 oder 1 steht und
- R¹: jeweils unabhängig ausgewählt ist aus den Resten: C₁-C₄-Alkyl, C₁-C₄-Alkoxy, C₁-C₄-Alkylthio, C₅-C₁₄-Aryl, C₅-C₁₄-Aryloxy, C₆-C₁₅-Arylalkyl, C₆-C₁₅-Arylalkoxy, Chlor, Fluor, Cyano, freies oder geschütztes Formyl, freies oder geschütztes Hydroxy, freies oder geschütztes Mercapto oder C₁-C₄-Halogenalkyl.

Eine besonders bevorzugte Verbindung der Formel (I) ist 1-(3-Nitrophenyl)ethylamin, wobei das (S)- Enantiomer noch weiter bevorzugt ist.

Die gleichen Vorzugsbereiche gelten analog für Verbindungen der Formel (Ib). Besonders bevorzugte Verbindungen der Formel (Ib) sind (R)- und (S)-1-(3-Nitrophenyl)ethylamin-Hydrochlorid, wobei das (S)- Enantiomer noch weiter bevorzugt ist.

Die eneatiomerenangereicherten Verbindungen der Formel (Ib) sind von der Erfindung ebenfalls umfasst.

Die Umsetzung der Verbindungen der Formel (III) mit den Verbindungen der Formel (IVa) oder (IVb) zu Verbindungen der Formel (V) erfolgt bevorzugt in einem organischen Lösungsmittel wie insbesondere aliphatischen Alkoholen. Besonders bevorzugt ist Ethanol.

Bevorzugte Verbindungen der Formel (IVa) sind: Hydroxylamin und O-Methylhydroxylamin, wobei O-Methylhydroxylamin besonders bevorzugt ist.

Bevorzugte Verbindungen der Formel (IVb) sind: Hydroxylamin-Hydrochlorid und O-Methylhydroxylamin-Hydrochlorid, wobei O-Methylhydroxylamin-Hydrochlorid besonders bevorzugt ist.

Die Reaktionstemperatur in Schritt A) kann beispielsweise 0°C bis zum Siedepunkt des verwendeten Lösungsmittels betragen, bevorzugt ist eine Umsetzung bei 10 bis 100°C, besonders bevorzugt ist eine Umsetzung bei 70 bis 80°C.

Die Reduktion im Schritt B) findet in Gegenwart von
- organischen Lösungsmittel
- mit einem komplexen Borhydrid und
- einer Säure statt.

Bevorzugte organische Lösungsmittel für Schritt B) sind Ether wie Alkylenglykolalkylether wie Dipropylenglykolmonomethylether, Diethylenglykolmono-butylether, Diethylether, Dioxan, Tetrahydrofuran und tert.-Methyl-butylether gegebenenfalls in Mischung mit aromatische und/oder aliphatische Kohlenwasserstoffen.

Bevorzugte komplexe Borhydride sind solche der Formel (VI)

Met[BH_{q}R⁶ _{(4-q)}]ₚ (VI)

in der
- Met: für ein ein- oder zweiwertiges Metall wie vorzugsweise Zink, Lithium, Natrium oder Kalium steht und
- R⁶: für C₁-C₈-Alkyl steht und
- q: für 1, 2, 3 oder 4, bevorzugt für 4 oder 1 und
- p: für die Wertigkeit von Met steht.

Besonders bevorzugt ist Natriumborhydrid.

Schritt B) findet weiterhin in Gegenwart von Säure statt. Dabei umfasst der Begriff Säure Lewissäuren wie beispielsweise Titan-, Zirkon- und Borhalogenide, als auch Brönstedt-Säuren wie insbesondere Carbonsäuren, und auch Verbindungen die bei Reaktion mit komplexen Borhydriden Lewis- oder Brönstedt-Säuren bilden wie beispielsweise Iod oder Di(C₁-C₄)alkylsulfate.

Besonders bevorzugt sind Carbonsäuren wie Trifluoressigsäure und Borhalogenide wie Bortrifluorid, wobei Bortrifluorid ganz besonders bevorzugt ist.

Gemäß Schritt B) werden racemische Verbindungen der Formel (Ia) erhalten.

In einer bevorzugten Ausführungsform erfolgt die Aufarbeitung in Schritt B) durch Versetzen mit einer wässrigen Säure oder Wasser und Säure auf einen pH-Wert von <5 (bezogen auf Raumtemperatur) und Extraktion der wässrigen Lösung mit einem organischen Lösungsmittel.

Als Säuren können anorganische oder organische Säuren verwendet werden, bevorzugt sind anorganische Säuren, besonders bevorzugt ist Salzsäure.

Als Extraktionsmittel können organische Lösungsmittel verwendet werden. Bevorzugt sind Alkylether, Arylether, aliphatische Kohlenwasserstoffe, aromatische Kohlenwasserstoffe und halogenierte Kohlenwasserstoffe.

Bevorzugt sind Alkylether, Arylether und halogenierte Kohlenwasserstoffe. Besonders bevorzugt ist Dichlormethan.

Die Freisetzung der Verbindung der Formel (Ia) aus der wässrigen Lösung kann dann beispielsweise durch Versetzen mit einer Base auf einen pH-Wert von >8 und Extraktion mit einem organischen Lösungsmittel erfolgen.

Als Base können beispielsweise und bevorzugt Alkali- oder Erdalkali-hydroxide und -carbonate oder organische Basen eingesetzt werden. Bevorzugt Alkali- oder Erdalkali-hydroxide. Besonders bevorzugt ist Natriumhydroxid.

Die Extraktion der Verbindung der Formel (Ia) kann mit Hilfe eines organischen Lösungsmittels erfolgen. Bevorzugt sind Alkylether, Arylether, aliphatische Kohlenwasserstoffe, aromatische Kohlenwasserstoffe und halogenierte Kohlenwasserstoffe. Bevorzugt sind Alkylether, Arylether und halogenierte Kohlenwasserstoffe. Besonders bevorzugt ist Dichlormethan.

Im Schritt C) erfolgt Enantiomerenanreicherung mit Hilfe von enantiomerenangereicherten Säuren.

Bevorzugt sind enantiomerenangereicherte Carbon- oder Sulfonsäuren, besonders bevorzugt ist Camphersulfonsäure.

Der Begriff "enantiomerenangereicherte Carbon- oder Sulfonsäuren" bedeutet im Sinne dieser Erfindung die jeweiligen enantiomerenreinen Carbon- oder Sulfonsäuren oder Mischungen der jeweiligen Enantiomere, in denen ein Enantiomer in einem Enantiomerenüberschuss, im Folgenden auch ee ("enantiomeric excess") genannt, im Vergleich zum anderen Enantiomer vorliegt. Bevorzugt beträgt dieser ee-Wert 85 bis 100 %, besonders bevorzugt 95 bis 100 % und ganz besonders bevorzugt 98 bis 100 %.

In allen anderen Zusammenhängen bedeutet der Begriff "enantiomerenangereichert" enantiomerenreine Stoffe (ee > 99,5 %) oder Mischungen der jeweiligen Enantiomere, in denen ein Enantiomer in einem Enantiomerenüberschuss im Vergleich zum anderen Enantiomer vorliegt, wobei dieser ee-Wert bevorzugt 10 bis 100 %, besonders bevorzugt 50 bis 100 % und ganz besonders bevorzugt 70 bis 100 % beträgt.

Gemäß Schritt C) werden dann zunächst die diastereomeren Verbindungen der Formel (Ic) erhalten 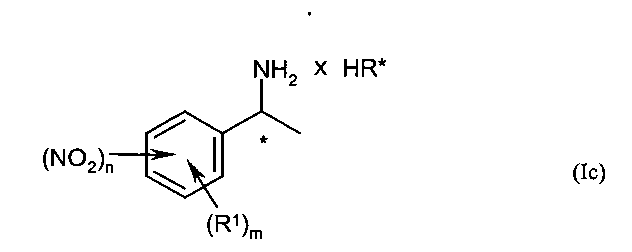 in der R* für das Anion einer enantiomerenangereicherten organischen Säure steht.

Die diastereomeren Verbindungen der Formel (Ic) lassen sich aufgrund ihrer unterschiedlichen physikalischen Daten fraktioniert kristallisieren.

Die Verbindungen der Formel (Ic) sind in diastereomerenangereicherter Form von der Erfindung als wichtige Intermediate ebenfalls umfasst.

Die Freisetzung der nitrosubstituierten, enantiomerenangereicherten 1-Phenylethylamine der Formel (I) aus den diastereomerenangereicherten Verbindungen der Formel (Ic) kann durch Versetzen mit Base und anschließende Extraktion mit organischem Lösungsmittel erfolgen, wobei für den Begriff Base und organisches Lösungsmittel die gleichen unter Schritt B) genannten Vorzugsbereiche gelten.

Um Oxidation der freien Verbindungen der Formel (I) zu vermeiden, kann vorzugsweise anschließend im Schritt D) die Überführung in Verbindungen der Formel (Ib) erfolgen.

Auf erfindungsgemäße Weise können enantiomerenangereicherte, nitrosubstituierte 1-Phenylethylamine der Formel (I) oder die entsprechenden Ammoniumsalze der Formel (Ib) in hohen Ausbeuten und ee-Werten von bis zu 99,7 erhalten werden.

Die erfindungsgemäßen nitrosubstituierten, enantiomerenangereicherte 1-Phenylethylamine eignen sich insbesondere in einem Verfahren zur Herstellung von pharmazeutischen Wirkstoffen wie beispielsweise IMPDH-Inhibitoren (siehe auch WO-A 00/56331).

### Beispiele

### Beispiel 1

### Herstellung von 3-Nitro-N-methoxyiminoacetophenon

240,0 g (1,453 mol) 3-Nitroacetophenon werden bei Raumtemperatur in 1200 ml Ethanol suspendiert. Zu dieser Suspension wird eine Lösung aus 132,0 O-Methylhydroxylamin-Hydrochlorid in 120 ml Wasser in 45 min zugetropft. Das Reaktionsgemisch wird zum Sieden erhitzt und 4h bei dieser Temperatur gerührt. Die Suspension wird anschließend heiß filtriert. Das Filtrat wird über einen Zeitraum von 12h auf Raumtemperatur abgekühlt. Der dabei ausgefallene farblose Feststoff wird über eine Fritte abgesaugt und dreimal mit je 100 ml Ethanol gewaschen. Das Produkt wird bei 50 °C und einem Druck von 100 mbar über einen Zeitraum von 2h getrocknet. Es werden 235,3 g (83,2 % d. Th.) eines leicht gelben Feststoffes erhalten.

### Beispiel 2

### Herstellung von 1-(3-Nitrophenyl)ethylamin

### a) Aktivierung mit Essigsäure

100 g trockenes Tetrahydrofuran werden unter einer Argonatmosphäre vorgelegt. Zu dem Lösungsmittel werden 3,78 g (0,10 mol) Natriumborhydrid zugegeben. Die Suspension wird 15 min bei Raumtemperatur gerührt. Anschließend werden 6,0 g (0,10 mol) Essigsäure bei einer Temperatur von 20°C zugetropft, wobei starke Gasentwicklung eintritt. Über einen Zeitraum von 20 min wird dann eine Lösung aus 4,86 g (0,025 mol) 3-Nitro-*N*-methoxyiminoacetophenon in 40 ml Tetrahydrofuran zugegeben. Das Reaktionsgemisch wird 2h bei Raumtemperatur gerührt und anschließend 2 h am Rückfluss erhitzt. Danach wird der Ansatz auf 10°C gekühlt und bei dieser Temperatur unter Rühren 60 ml Wasser zugetropft. Das Lösungsmittel wird am Rotationsverdampfer entfernt. Der Rückstand wird in 150 ml Wasser aufgenommen und mit 10 ml konz. Salzsäure auf pH 1 gestellt. Die wässrige Lösung wird mit 50 ml Dichlormethan gewaschen. Anschließend wird die wässrige Phase mit 4,5 ml 45 %iger Natronlauge auf pH 9 gestellt und zweimal mit je 25 ml Dichlormethan extrahiert. Die vereinigten organischen Phasen werden über Natriumsulfat getrocknet. Das Lösungsmittel wird am Rotationsverdampfer entfernt. Es werden 0,48g (7,5 % d. Th.) des Produktes als oranges Öl erhalten.

### b) Aktivierung mit Trifluoressigsäure

500 g trockenes Tetrahydrofuran werden unter einer Stickstoffatmosphäre vorgelegt. Zu dem Lösungsmittel werden 37,8 g (1,00 mol) Natriumborhydrid gegeben. Die Suspension wird 15 min bei Raumtemperatur gerührt. Anschließend werden 114 g (1,00 mol) Trifluoressigsäure bei einer Temperatur von 20°C zugetropft, wobei starke Gasentwicklung eintrat. Über einen Zeitraum von 45 min wird dann eine Lösung aus 48,6 g (0,25 mol) 3-Nitro-*N*-methoxyiminoacetophenon in 200 ml trockenem Tetrahydrofuran zugegeben. Das Reaktionsgemisch wird 2 h bei Raumtemperatur gerührt und anschließend 2h auf 50°C erhitzt. Danach wird der Ansatz bei 50°C über einen Zeitraum von 35 Minuten in 400 ml Wasser unter Eiskühlung dosiert. Das Reaktionsgemisch wird anschließend auf 50°C geheizt und bei dieser Temperatur 2h gerührt. Danach wird der Ansatz auf Raumtemperatur gekühlt und mit 130 ml konz. Salzsäure auf pH 1 gestellt. Das Lösungsmittel wird am Rotationsverdampfer bis zu einer Sumpftemperatur von 50°C bei einem Druck von 100 mbar abdestilliert. Zu dem Sumpf werden 500 ml Toluol gegeben. Das Reaktionsgemisch wird filtriert. Die organische Phase wird abgetrennt. Die wässrige Phase wird mit 500 ml Toluol gewaschen. Die organischen Phasen werden verworfen. Die wässrige Phase wird mit 60 ml 45 %iger Natronlauge auf pH 10 gestellt und zweimal mit je 500 ml Toluol extrahiert. Das Lösungsmittel der vereinigten organischen Phasen wird am Rotationsverdampfer entfernt. Es werden 27,6 g (58,2 % d. Th.) des Produktes als oranges Öl erhalten.

### c) Aktivierung mit Bortrifluorid-Diethylether-Komplex

106 g trockenes Tetrahydrofuran werden unter einer Stickstoffatmosphäre vorgelegt. Zu dem Lösungsmittel werden 61,4 g (1,54 mol) Natriumborhydrid gegeben. Die Suspension wird 15 min bei Raumtemperatur gerührt. Über einen Zeitraum von 35 min wird dann eine Lösung aus 100,0g (0,51 mol) 3-Nitro-*N*-methoxyiminoacetophenon und 501 g trockenem Tetrahydrofuran in 228g Toluol zugegeben. Anschließend werden 219 g (1,54 mol) Bortrifluorid-Diethylether-Komplex bei einer Temperatur von 20°C innerhalb von 30 min zugetropft, wobei starke Gasentwicklung eintrat. Das Reaktionsgemisch wird 5 h bei Raumtemperatur gerührt und anschließend 2 h auf 40°C erhitzt. Danach wird der Ansatz über einen Zeitraum von 4 h in 1200 ml Wasser unter Eiskühlung dosiert. Das Reaktionsgemisch wird anschließend bei 20°C 10 min gerührt. Danach wird der Ansatz mit 107 ml konz. Salzsäure auf pH 1 gestellt, auf 50°C erwärmt und 2 h nachgerührt Die Phasen werden getrennt. Die organische Phase wird verworfen. Die wässrige Phase wird mit 500 ml Dichlormethan gewaschen. Die organischen Phase wird verworfen. Die wässrige Phase wird filtriert, mit 140 ml 45 %iger Natronlauge auf pH 10 gestellt und dreimal mit je 500 ml Dichlormethan extrahiert. Das Lösungsmittel der vereinigten organischen Phasen wird am Rotationsverdampfer entfernt. Es werden 66,3 g (78,3 % d. Th.) des Produktes als oranges Öl erhalten.

### Beispiel 3

### Herstellung von (S)-1-(3-Nitrophenyl)ethylamin

### a) Kristallisation mit L-(+)-Weinsäure

0,75 g (5,0 mmol) L-(+)-Weinsäure werden in 64 ml Methanol gelöst und die Lösung auf Rückfluss erhitzt. Eine Lösung aus 1,0 g (6,0 mmol) 1-(3-Nitrophenyl)ethylamin aus Beispiel 2 in 7,1 ml Methanol wird über einen Zeitraum von 5 min zudosiert und der Ansatz anschließend 15 min unter Rückfluss gerührt. Die Lösung wird über einen Zeitraum von 40 min auf 50°C und anschließend über einen Zeitraum von 1,5 h auf 35°C abgekühlt. Die Lösung wird daraufhin 4 Tage bei 5°C gelagert. Der dabei ausgefallene Feststoff wird abfiltriert. Es werden 0,5 g eines beigen Feststoffes erhalten. Dieser wird in 10 ml Wasser suspendiert und mit 0,9 ml 10 %iger Natronlauge auf pH 10 gestellt. Die Lösung wird zweimal mit je 30 ml Dichlormethan extrahiert. Das Lösungsmittel der vereinigten organischen Phasen wird am Rotationsverdampfer entfernt. Es werden 0,3 g (30 % d. Th.) (*S*)-1-(3-Nitrophenyl)ethylamin in Form eines braunen Öles erhalten. Der ee-Wert beträgt 70 %.

### b) Kristallisation mit (+)-Camphersulfonsäure

226,7 g (1,364 mol) 1-(3-Nitrophenyl)ethylamin aus Beispiel 2 werden bei 22°C in 2830 ml Wasser gegeben. Über einen Zeitraum von 5 min werden 301,0 g (1,296 mol) (+)-Camphersulfonsäure zugegeben. Dabei steigt die Temperatur auf 30°C an. Das Reaktionsgemisch wird auf 60°C erwärmt und 10 min bei dieser Temperatur gerührt. Der Ansatz wird dann über einen Zeitraum von 18 h auf 10°C gekühlt. Der ausgefallene Feststoff wird abgesaugt und viermal mit je 100 ml Wasser nachgewaschen. Es werden 224,5 g eines beigen Produktes erhalten. Der ee-Wert beträgt 92,6 %.

222,0 g des erhaltenen Feststoffes werden in 500 ml Wasser suspendiert. Die Suspension wird auf 95°C erhitzt und 10 min bei dieser Temperatur gerührt. Die Lösung wird über einen Zeitraum von 18 h auf 10°C gekühlt. Der ausgefallene Feststoff wird abgesaugt und dreimal mit je 100 ml Wasser nachgewaschen. Der Feststoff wird 1h bei einem Druck von 100 mbar getrocknet. Es werden 128,7 g (Insgesamt 23,7 % d. Th.) eines beigen Feststoffes erhalten. Der ee-Wert beträgt 99,7 %.

### Beispiel 4

### Herstellung von (S)-1-(3-Nitrophenyl)ethylamin-Hydrochlorid

126,5 g (317 mmol) des so erhaltenen (S)-1-(3-Nitrophenyl)ethylamin-(+)-camphersulfonates werden in 200 ml 2N Natronlauge gelöst. Zu der Lösung werden 100 ml Wasser gegeben. Die Phasen werden getrennt. Die wässrige Phase wird zweimal mit je 100 ml Dichlormethan extrahiert. Das Lösungsmittel der vereinigten organischen Phasen wird am Rotationsverdampfer entfernt. Zum Rückstand werden 100 ml Dichlormethan und 5g Aktivkohle gegeben. Die Lösung wird über Celite filtriert. Der Filterrückstand wird mit 50 ml Dichlormethan nachgewaschen.

Durch das Filtrat wird während 20 min Chlorwasserstoffgas geleitet. Durch die Suspension wird anschließend 3 min Stickstoff geleitet. Der Feststoff wird abgesaugt und zweimal mit je 50 ml Dichlormethan gewaschen. Nach dem Trocknen des Feststoffes bei 40°C und einem Druck von 100 mbar werden 53,0 g (100 % d. Th.) eines beigen Produktes erhalten. Der ee-Wert beträgt 99,6 %.

## Patentansprüche

1. Verfahren zur Herstellung von enantiomerenangereicherten Verbindungen der Formel (I) 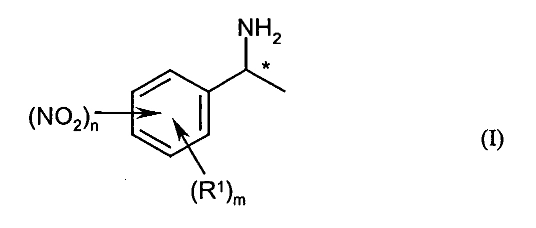 in der
* ein stereogenes Kohlenstoffatom markiert
n für 1,2 oder 3 steht,
m für eine ganze Zahl zwischen 0 und (5-n) steht und
R¹ jeweils unabhängig ausgewählt ist aus den Resten: C₁-C₁₂-Alkyl, C₁-C₁₂-Alkoxy, C₁-C₁₂-Alkylthio, C₅-C₁₄-Aryl, C₅-C₁₄-Aryloxy, C₆-C₁₅-Arylalkyl, C₆-C₁₅-Arylalkoxy, Chlor, Fluor, Cyano, freies oder geschütztes Formyl, freies oder geschütztes Hydroxy, freies oder geschütztes Mercapto, C₁-C₁₂-Halogenalkyl, C₁-C₁₂-Halogenalkylthio, C₁-C₁₂-Halogenalkoxy und Resten der Formeln (IIa) und (IIb),
A-B-D-E (IIa)
A-E (IIb)
in denen
A fehlt oder für einen C₁-C₈-Alkylen-, C₁-C₈-Alkenylen- oder C₁-C₈-Halogenalkylenrest steht und
B fehlt oder für Sauerstoff, Schwefel oder NR² steht, wobei
R² für Wasserstoff, C₁-C₈-Alkyl, C₆-C₁₅-Arylalkyl oder C₅-C₁₄-Aryl steht und
D für eine Carbonyl-Gruppe steht und
E für OR³ oder N(R⁴)₂ steht, wobei
R³ für C₁-C₈-Alkyl, C₆-C₁₅-Arylalkyl oder C₅-C₁₄-Aryl und
R⁴ jeweils unabhängig für Wasserstoff, C₁-C₈-Alkyl, C₆-C₁₅-Arylalkyl oder C₆-C₁₄-Aryl steht oder N(R⁴)₂ zusammen für einen cyclischen Aminorest mit 4 bis 12 Kohlenstoffatomen steht und
**dadurch gekennzeichnet, dass**
• in einem Schritt A)
Verbindungen der Formel (III) 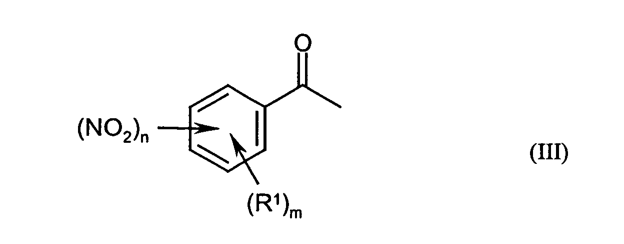 mit Verbindungen der Formel (IVa) oder (IVb)
R⁵O-NH₂ (IVa)
R⁵O-NH₂ • HX (IVb)
in denen
R⁵ für Wasserstoff oder C₁-C₁₂-Alkyl steht und
X für ein Anion steht
zunächst zu Verbindungen der Formel (V) 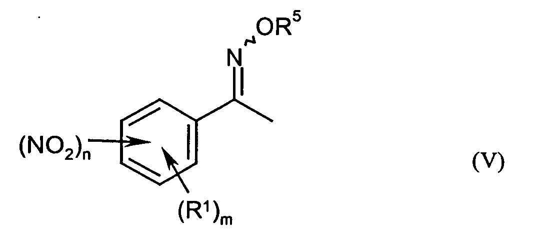 umgesetzt werden und
• in einem Schritt B)
die Verbindungen der Formel (V)
- in einem organischen Lösungsmittel
- mit einem komplexen Borhydrid und
- einer Säure
zu racemischen Verbindungen der Formel (Ia) 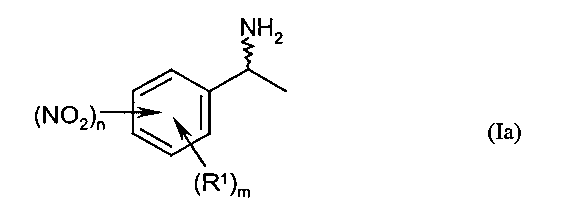 reduziert werden und
in einem Schritt C)
die racemischen Verbindungen der Formel (Ia) mit Hilfe von enantiomerenangereicherten Säuren enantiomerenangereichert werden.

2. Verfahren nach Anspruch 1, **dadurch gekennzeichnet, dass** als weiterer Schritt D) die Umsetzung der enantiomerenangereicherten Verbindungen der Formel (I) zu enantiomerenangereicherten Ammoniumsalzen der Formel (Ib) erfolgt 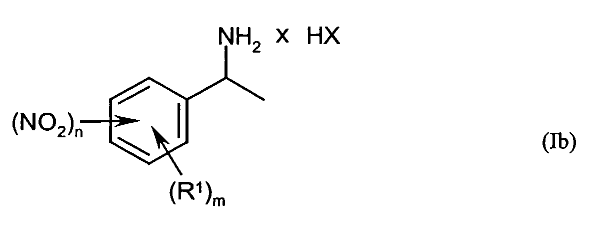 in der X für ein Halogenid oder ein Sulfonat steht.

3. Verfahren nach einem oder mehreren der Ansprüche 1 bis 2, **dadurch gekennzeichnet, dass**
n für 1,
m für 0 oder 1 steht und
R¹ jeweils unabhängig ausgewählt ist aus den Resten: C₁-C₄-Alkyl, C₁-C₄-Alkoxy, C₁-C₄-Alkylthio, C₅-C₁₄-Aryl, C₅-C₁₄-Aryloxy, C₆-C₁₅-Arylalkyl, C₆-C₁₅-Arylalkoxy, Chlor, Fluor, Cyano, freies oder geschütztes Formyl, freies oder geschütztes Hydroxy, freies oder geschütztes Mercapto oder C₁-C₄-Halogenalkyl.

4. Verfahren nach einem oder mehreren der Ansprüche 1 bis 3, **dadurch gekennzeichnet, dass** 1-(3-Nitrophenyl)ethylamin bzw. Nitrophenyl)ethylamin-Hydrochlorid hergestellt wird.

5. Verfahren nach einem oder mehreren der Ansprüche 1 bis 4, **dadurch gekennzeichnet, dass** in Schritt B) als komplexe Borhydride solche der Formel (VI) eingesetzt werden
Met[BH_{q}R⁶ _{(4-q)}]ₚ (VI)
in der
Met für ein ein- oder zweiwertiges Metall wie vorzugsweise Zink, Lithium, Natrium oder Kalium steht und
R⁶ für C₁-C₈-Alkyl steht und
q für 1, 2, 3 oder 4, bevorzugt für 4 oder 1 und
p für die Wertigkeit von Met steht.

6. Verfahren nach einem oder mehreren der Ansprüche 1 bis 5, **dadurch gekennzeichnet, dass** im Schritt B) als Säure Lewissäuren, Brönstedt-Säuren und Verbindungen eingesetzt werden, die bei Reaktion mit komplexen Borhydriden Lewis- oder Brönstedt-Säuren bilden.

7. Verfahren nach einem oder mehreren der Ansprüche 1 bis 2, **dadurch gekennzeichnet, dass** im Schritt C) Camphersulfonsäure eingesetzt wird.

8. Diastereomerenangereicherte Verbindungen der Formel (Ic) 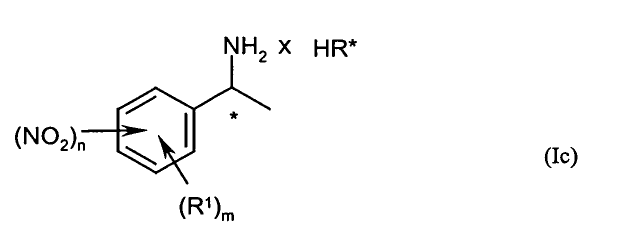 in der n,m und R¹ die in Anspruch 1 angegebene Bedeutung besitzen und R* für das Anion einer enantiomerenangereicherten organischen Säure steht.

9. (*S*)*-*1-(3-Nitrophenyl)ethylammonium-Camphersulfonat.

10. Enantiomerenangereicherte Verbindungen der Formel (Ib) 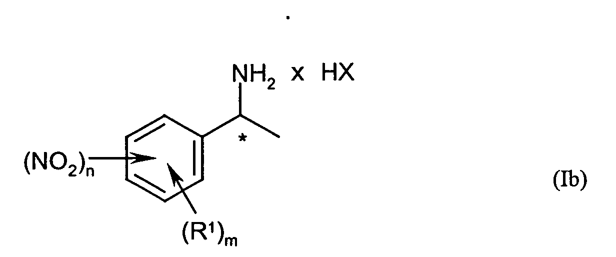 in der n, m und R¹ die in Anspruch 1 angegebene Bedeutung besitzen und X für ein Halogenid steht.

11. (*S*)-1-(3-Nitrophenyl)ethylammonium-chlorid.

12. Verwendung von Verbindungen gemäß Ansprüchen 8 und 9 oder 10 und 11 sowie Verbindungen die nach einem oder mehreren der Ansprüche 1 bis 7 hergestellt wurden zur Herstellung von pharmazeutischen Wirkstoffen.
